(19) Europäisches Patentamt

European Patent Office

Office européen des brevets

(11) **EP 1 422 292 A1**

(12) **EUROPEAN PATENT APPLICATION**
published in accordance with Art. 158(3) EPC

(43) Date of publication:
26.05.2004 Bulletin 2004/22

(51) Int Cl.⁷: **C12N 1/14**, A61K 35/84
// C12N1:14, C12R1:645

(21) Application number: 02752960.1

(22) Date of filing: 22.07.2002

(86) International application number:
**PCT/CN2002/000508**

(87) International publication number:
**WO 2003/025155 (27.03.2003 Gazette 2003/13)**

(84) Designated Contracting States:
**AT BE BG CH CY CZ DE DK EE ES FI FR GB GR
IE IT LI LU MC NL PT SE SK TR**

(30) Priority: **17.08.2001 CN 01126498**

(71) Applicant: **Hangzhou Tsbio Science &
Technology Co., Ltd.
Hangzhou, Zhejiang 310029 (CN)**

(72) Inventors:
• **GUO, Xinjun
Hangzhou, Zhejiang 310029 (CN)**
• **KE, Chuankui
Hangzhou, Zhejiang 310011 (CN)**

(74) Representative: **Schrell, Andreas, Dr. et al
Gleiss & Grosse
Leitzstrasse 45
70469 Stuttgart (DE)**

(54) **FERMENTATION PRODUCT OF CYPTOPORUS VOLVATUS AND ITS PREPARATION METHOD AND USE**

(57) This invention provides a preparation method of fermentation product of Cryptoporus volvatus (Peck) Schear and its clinical use, the said Cryptoporus volvatus (Peck) Schear is cultured on culture medium for fluid fermentation or solid fermentation to obtain mycelium of Cryptoporus volvatus (Peck) Schear, culture supernatant fluid and various metabolins, the culture medium of which contains glucose, maltose, yeast powder, peptone, inorganic salts and vitamins. The said fermentation product can be made into kinds of drug forms to prevent and treat irritability diseases, for example, bronchus asthma, allergic rhinitis, allergic gastroenteritis or ophthalmopathy which is caused by pollen, acarid, mildew or dust. Moreover, it can be used for preventing and treating cough.

EP 1 422 292 A1

**Description**

Field of the Invention

The present invention relates to a kind of fermentation product of Cryptoporus volvatus (Peck) Schear, its preparation method, and pharmaceutical application.

Background of the Invention

Cryptoporus volvatus (Peck) Schear, also named as Songganlan, Shugeda, Hesejun, Muyujun, Xiangmulan and Yikouer in Chinese, is classified into Cryptoporus, Polyporaceae, Aphyllophorales, Hymenomycetes, and Basidiomycotina. Cryptoporus volvatus (Peck) Schear is a wood decaying fungus and grows on the shadow side of withered branches of pine trees or earthward side of fallen trees. The parts being used as therapeutic medication are fresh or dry sporophore, mycelium and metabolin of submerged culture. It is distributed in North Korea, Japan, Europe, North America and many China provinces, including Hebei, Sichuan, Yunnan, Jiangsu, Hubei, Guangxi, Fujian, Hainan and Jilin etc. Cryptoporus volvatus (Peck) Schear contains polysaccharide- proteins, ergosterols, quite bitter sesquiterpenoids and aromatics; its wild sporophore has been recorded in pharmacological references. LanMao (1397-1476) described in *Materia Medica of Diannan* "Songganlan tastes bittersweet, with slightly cool feature. It is used for treatment of disorders such as intestine bleeding, toxic heat and many kinds of hemorrhoids. When it is bitten by painful teeth, the pain disappears". It is recorded in *Illustrated Handbook of Chinese Pharmaceutical Fungi (by Ying Jianzhe etc. 1987)* that Cryptoporus volvatus (Peck) Schear can be used to treat tracheitis and asthma. Moreover, it can diminish inflammation". *Chinese Materia Medica* records that it tastes slightly bitter, with mild feature. It has the effects of stopping cough, subduing asthma and detoxification and is used for treating tracheitis and asthma". Li et al of South Korea reported in 1981 that extract of Cryptoporus volvatus (Peck) Schear by hot water contains polysaccharide-proteins which has anticancer activity. In 1988, seven sesquiterpenoids with quite bitter taste, namely, cryptoporic acid A, B, C, D, E, F and G, were separated and purified from carposporophyte of Cryptoporus volvatus (Peck) Schear by Hashimoto Toshihiro et al. and Asakawa Yoshinori et al. of Japan. Experiment in rats showed that cryptoporic acid C, E, F and G had strong inhibitory activity on oxygen radicals, which were released from abdominal macrophage when being stimulated. The plant morphology, entironment, biological characteristics and pharmaceutical effects of Cryptoporus volvatus (Peck) Schear have been reported in domestic and foreign journals of literatures, such as *Study On Biological Characteristics Of Cryptoporus Volvatus (Peck) Schear As A Pharmaceutical Fungus (by Hua Qihong, Sun Jing, and Shen Li, China Journal of Traditional Chinese Medicine, 1991, Vol 16, No. 12)*. In 2000, study of M. Zang et al. showed that cryptoporus volvatus (Peck) Schear distributed in China mostly was cryptoporus sinensis Sheng H. Wu & M. Zang. Because of rigorous ecological environment required by Cryptoporus volvatus (Peck) Schear, the difficulty in collecting sporophore, the shortness of storage, the lack of resource, and the great difficulties in artificially culturing sporophore, the pharmaceutical value of this fungus has not been implicated to therapeutic application and medicinal materials standard concerning this fungus has not yet been established.

Long before, people had realized that after contacting certain substances, organism would generate some egregious reactions, such as edema, dermal itch, asthma etc. symptoms and even lead to death. In 1819, John Bostock described pollinosis firstly. In 1873, Charles Blokley found that pollen was the pathogeny of pollinosis. In 1906, the Austrian doctor Clemens vo Pirquet presented the term——Allergy when he found that after receiving serum treatment, some patients , instead of being cured, took on the symptom of hyperpyrexia, systemic lymphadenonectasis, articular pain, hepatosplenomegaly and renal failure. In 1921, Prausnitz and Kustner discovered there was an anaphylactic factor in vivo of irritability diseases, which could be transferred to healthy human's tissues and lead to allergy. In 1963, P. C. H. Cell and R. R. A. Coombs classified allergy into 4 types, which is more reasonable. In 1966, the Japanese American couple Kimishige Ishizaka and Teruko Ishizaka discovered the antibody IgE of immediate allergy for the first time. At present, allergy has become an independent subject and the concept of chronic anaphylactic inflammation enhances people's cognition on I type allergy.

Investigation and statistics of epidemiology indicate that in various allergic diseases, incidence of anaphylactic dermatosis is the highest and accounts for about 44%. Wherein, the most frequent diseases include hives, angioneurotic edema, afodic dermatitis, contact dermatitis and drug eruption. 11% of allergic patients are various respiratory anaphylaxis patients, wherein bronchial patients occupy 4.6% and allergic rhinitis patients occupy 6.7%. And 3.2% are drug allergy patients; 1%-2% are gastrointestinal allergy patients; about 5% are other various unusual allergic diseases patients. During the past thirty years, with economic development and improvement of living standard, allergic diseases obviously have been increasing all over the world.

Allergic diseases mainly involve five aspects i.e. allergen, antibody, cell, receptor and medium. Anaphylactic substances, such as pollen, dust, food, fungi, drug, scurf of human or animal, feather, insects, parasites and other chemicals, cause allergy of organism via inhalation, eating, injection and contact, then induce B cell to produce antibody IgE which binds with receptor of surface of target cell such as mast cell and eosinophil etc. When organism contacts the same antigen again, the latter will have special reaction to the IgE antibody, which binds target cells. It is possible to have bridging reaction when the specific antigen binds more than two IgE molecules. Bivalent or multi-valent antigen molecule approaches more than two IgE molecules and changes its structure, then induces assembly of IgE receptors and degranulation of medium cell. After cell degranulation, certain enzymes and vasoactive substances are released, these may cause smooth muscle contraction, secretion of grume, decrease of blood pressure, and tissue injury. Moreover, allergen can be mediated by homocytotropic antibody IgE to activate eosinophils and release a lot of inflammatory factors, such as Leukotriene, Prostapar, Thromboxane A2 and cytokines, etc, that lead to inflammation of derma, bronchus mucosa, nasal mucosa and gastrointestinal mucosa.

Of various irritability diseases, pathogenesis of asthma has made great progress in recent several years. Asthma is a kind of chronic airway inflammation involving many inflammatory cells such as mast cell, eosinophil and T lymphocyte, etc. This inflammation makes susceptible show airway hyper reactivity to manifold stimulus and cause tracheostenosis, with symptoms of recurrent asthma, dyspnea, chest distress or cough. Paroxysm and intensification of these symptoms usually happen at night and/or early morning, and extensive and changeful reversible airflow limitation often occurs. Most patients can be relieved by themselves or by receiving treatment.

Pathogenesis of asthma associates with a chronic inflammatory process of airway walls. This process can cause airflow limitation and hyper-reactivity, thus airway becomes narrower when reacting to different stimulators. Airway inflammation is typically characterized by increase in number of active eosinophils, mast cells and T lymphocytes in respiratory mucosa and lumina, thickening of substratum of basement membrane and fibrosis of subepithelia. Studies indicate that there is a close connection between the increase of reversible airflow limitation characterized by asthma symptom and abnormal lung functions of asthma and the increase of inflammation reactivity. The factors that may cause asthma include: inclination factors, including diathesis and gender; pathogeny factors, including indoor allergen (including acarid, animal allergen, roach allergen and fungi), outdoor allergen (including pollen and fungi), aspirin, professional anaphylactic substance; promotion factors, including infection of respiratory tract, low birth weight, meal, air pollution (including outdoor pollutant and indoor pollutant), smoking (including active smoking and passive smoking).

In recent twenty years, incidence and mortality of asthma have gradually increased throughout the world. Data indicates that there are 150 million asthma patients in the world and 100 thousand patients die of asthma unnecessarily which should be avoided by nature. Asthma affects not only physical growth and health, but also psychological development, including self-respect. Asthma as a long-term disease will burden individual and society extensively, reduce quality of life or affect physical health, cause progressive loss of functions and early death, and will lower productivity and increase health expenses. Thus, asthma is a crucial problem of public health and should be paid attention to by government and department of public health.

As Asthma is a chronic airway inflammatory disease, the most effective treatment is to prevent inflammation by eliminating inducing factors. Medication of asthma is used to reverse and prevent symptoms and airflow limitation, including control drug and relief drug. Control drug is used for durative asthma, including anti-inflammation drug and long-effective bronchodilator. The former is the most effective control drug at present, such as corticoid, which can control asthma in one or more aspects but with serious side effects. The latter can dilate airway by relaxing airway smooth muscles. Although bronchodilator can reverse or restrain shrink of bronchus, it can't reverse airway inflammation and airway hyper-reactivity. At present, some drugs concerning leukotriene are being developed, such as antagonists of leukotriene, antagonists of a key enzyme of biosynthesis and antagonists of a lipid receptor on surface of smooth muscle cell or other cells which prevent the binding of lipids and smooth muscle cell, and therefore block out biologic activity of leukotriene.

Therefore, it is very significant to make use of pharmaceutical function of Cryptoporus volvatus (Peck) Schear and provide fermentation product of Cryptoporus volvatus (Peck) Schear as a drug to prevent and treat irritability diseases such as asthma.

Purpose of the Invention

The first purpose of the present invention is to provide a fermentation product of Cryptoporus volvatus (Peck) Schear in order to overcome the limitation of present drugs, such as serious side effects, disability to reverse airway inflammation and hyper-reactivity. The obtained fermentation product will be used to prevent and treat irritability diseases such as asthma. These drugs are highly effective, specialized, and are side-effect-free. It can reduce patients' dependence on steroid drugs, decrease dosage of steroid and improve life quality for patients.

The second purpose of the present invention is to provide preparation method of the above-mentioned fermentation product.

The third purpose of the present invention is to apply above fermentation product to prevent and treat irritability diseases such as asthma.

<u>Summary of the Invention</u>

In the present invention, fermentation product of Cryptoporus volvatus (Peck) Schear is defined as a mixture of mycelium and culture supernatant fluid. The dry-weight of this mixture is 5~50g/1000ml, wherein polysaccharide occupies 3~10%; volatile ether extract occupies 0.05~0.15%; total alkaloid occupies 0.03~0.25%. Analysis methods of polysaccharide, volatile ether extract and total alkaloid are described as below:

(1) Total polysaccharide

Take 10g powder of fermentation mixture, add in 200mL distilled water, boil and distill for 30min. After filtration, add another 100ml distilled water into the residue, and repeat the steps. Combine the filtrates of the two times, condense it properly, then dialyze it for three days in a dialysis bag with a molecular weight of 10,000 Dalton to remove small molecular substances in the solution. Then take it out and condense it to 10ml. Take a small amount of the condensed solution, and add in Molish reagent to make them react. There is purple or crimson circle on the interface of vitriol and water solution. This result indicates that the medicinal material contains polysaccharide.
Take a small amount of the condensed solution, and let it be electrophoresed in polyacrylamide gels and colorated by Schiff's reagent. There are continuous electrophoretic strips, which indicate the existence of polysaccharides with different molecular weights.
Take a small amount of the condensed solution; test its aniline blue deposition reaction. Blue deposit with infusibility in alcohol emerges, indicating the existence of $\beta$ -1,3 glucan.
Take 1ml of the condensed solution, dilute 200 times and measure its polysaccharide content by vitriol-phenol method. There are 30-100mg polysaccharide in per milliliter concentrated solution which means the fermentation mixture contains 3~10% polysaccharide.

(2) Volatile ether extract

Volatile oil extracted from plants has been used in clinic as medication to treat expectorant, diaphoresis, relieving cough, diuresis. It has the effects of sterilization or bacterium inhibition. The content of palmitic acid, which has the effects of anti-inflammation, bacterium inhibition, and analgesia, is comparatively higher in volatile oil of Cryptoporus volvatus (Peck) Schear. Sesquiterpenoids of volatile oil have functions of antibacterial, diminishing inflammation, analgesia and subduing asthma. It shows that volatile oil of Cryptoporus volvatus (Peck) Schear contains various effective components concerning clinic effect. Detailed method is described as follows: take 3g fermentation powders, weigh it accurately and put into a 50mL triangle bottle. Then add 20mL ether and shake for 12 hours. When the mixture goes steady, separate the ether layer and put it into an evaporating dish that has been dried to constant weight. Then add ether again until the ether extracting solution becomes colorless. Volatilize ether and dry for 18hours in vitriol desiccator. Weigh accurately, slowly heat to 105°C and dry to constant weight. The lost weight is the weight of volatile ether extract. The calculated results are shown in Table 1.

Table I

| Test on content of volatile ether extract | | | |
|---|---|---|---|
| Sample | 010302Y | 010306Y | 010307Y |
| Weight of sample (g) | 3.0039 | 3.0072 | 3.0023 |
| Weight after dried by vitriol (g) | 63.893 | 62.3398 | 60.2189 |
| Weight after heat to 105°C (g) 1 | 63.8892 | 62.3368 | 60.2154 |
| Weight after heat to 105°C (g) 2 | 63.8883 | 62.3345 | 60.2147 |
| Weight after heat to 105°C (g) 3 | 63.8886 | 62.3349 | 60.2152 |
| Content of volatile ether extract (%) | 0.14 | 0.146 | 0.127 |
| (010302Y, 010306Y, 010307Y are three batches of samples) | | | |

(3) Total alkaloids

Most of Alkaloids have biological activities of analgesia, are usually effective components of many Chinese traditional herbs and pharmaceutical plants, and can be used for relieving cough, subduing asthma, antibacterial, diminishing inflammation and relaxation of smooth muscles. In this invention, culture supernatant fluid is treated with ammonia solution to adjust pH>9, then extracted with chloroform many times. The total alkaloids are obtained by distilling off chloroform, and are examined by qualitative and quantitative tests.

Reactions to three deposition reagents are described as below:

Saffron yellow deposition emerges when reacting to potassium bismuth iodide solution; kelly deposition emerges quickly when reacting to phosphomolybdic acid; white deposition emerges quickly when reacting to silicotungstic acid (5%).

Thin Layer Chromatography (TLC) analysis:

Thin layer plate used in TLC test is silica gel GF254 plate (200mm x 50mm). Developing solvent is toluene-acetone-ethanol-concentrated ammonia solution (20:20:3:1). Well-separated spots with good reappearance are observed under ultra violet lamp.

The method of quantitative test on total alkaloids is acid dye colorimetry. The reference sample solution is prepared as follows: weigh 1mg morphine accurately, add 1mL methanol accurately, and then shake it up. Thus the reference sample solution is obtained (1mg morphine in 1mL sample).

The testing sample solution is prepared as follows: weigh 5g fermentation powder accurately and extract it for 24 hours with 40ml 1% $H_2SO_4$. Add concentrated ammonia solution to adjust pH to 9, then extract with ether for two times (40mL, 40mL) and extract with chloroform for two times (20mL, 20mL). Combine the organic solvent and reclaim it in warm water bath. Then add 20ml acid water solution (pH=3) accurately. The sample is obtained after the organic solvent is removed off. Protraction of standard curve: take accurately 20, 40, 60, 80, 100, 120 µl reference sample solution and put into 20mL separation funnels respectively. Add in pH3.0 acid water solution accurately, until the solution reaches 1ml. Add in 5mL chloroform and 1ml bromocresol green solution accurately (the mixture of 50mg bromocresol green and 1.021g potassium hydrogen phthalate is dissolved in 6.0ml 0.2mol/L sodium hydroxide solution and diluted to 100mL with water, then shaken up). Then shake the mixture acutely for 2min and keep statically for a while to separate the chloroform layer, and test absorbance at 420nm by spectrophotometry, protract standard curve.

Test of samples: take accurately 400 µl testing sample solution and 600 µl pH3.0 acid water solution, and put them into 20mL separation funnel. Add 5mL chloroform and 1mL bromocresol green solution accurately. Then shake acutely the mixture for 2min and keep statically for a while to separate the chloroform layer, test the absorbance at 420nm by spectrophotometry. The calculated results are shown in Table 2.

Table 2

| Contents of total alkaloids of samples | | | |
|---|---|---|---|
| Sample | 010302Y | 010306Y | 010307Y |
| Weight of sample (g) | 5.1367 | 5.1172 | 5.1284 |
| Content of sample (%) | 0.054 | 0.088 | 0.146 |

(010302Y, 010306Y, 010307Y are three batches of fermentation product of Cryptoporus volvatus (Peck) Schear)

This fermentation product is prepared as below:

(1) activation of strain: transplant the strain of Cryptoporus volvatus (Peck) Schear, which is kept at 4°C, to the following culture medium via asepsis operation, and culture aseptically for 3-10 days at normal culture temperature of fungi.

Ingredients and weight contents (%) of culture medium:

| | |
|---|---|
| glucose | 0.5-0.9 |
| maltose | 0.5-2 |
| peptone | 0.1-0.2 |
| yeast powder | 0.5-1 |
| $KH_2PO_4$ | 0.2-0.4 |
| $MgSO_4 \cdot 7H_2O$ | 0.2-0.5 |
| vitamin $B_1$ | 0.05-0.1 |
| agar | 2.2-3.0 |
| pH | 4.5-5.0 |

The rest is water.

(2) Shaking-culture: move above-mentioned activated Cryptoporus volvatus (Peck) Schear mycelial to a 500mL triangle flask containing 150-200mL culture fluid as follows. Shaking-culture the mixture for 6 days on shaking bed at 20°C-30°C and a shaking frequency of 100-200rpm. If you want to increase quantity, you can move mycelial solution to a 3000mL triangle flask containing 750mL culture fluid described as below. Shaking-culture the mixture for 1-2 days on shaking bed at 20°C-30°C and a shaking frequency of 100-200rpm.

Ingredients and weight contents (%) of culture fluid:

| glucose | 0.5-0.9 |
|---|---|
| maltose | 0.5-2 |
| peptone | 0.1-0.2 |
| yeast powder | 0.5-1 |
| $KH_2PO_4$ | 0.2-0.4 |
| $MgSO_4 \cdot 7H20$ | 0.2-0.5 |
| vitamin $B_1$ | 0.05-0.1 |
| pH | 4.5—5.0 |

The rest is water.

(3) Liquid fermentation culture: Put the culture fluid described below into fermenter of different capacities, the fluid covering 70% or less of the fermenter capacity. By method of pressure-margin, put in some of the shaking cultured Cryptoporus volvatus (Peck) Schear mycelial liquid, which is 3-5% of fermenter capacity, and ferment for 1-10 days at a ventilation quantity of 1: 0.1-1.0 (v/v/min), a impeller stirring speed of 100-200rpm, a fermenter pressure of more than $0.05kg/cm^2$ and a temperature of 20-30°C.

Ingredients and weight contents (%) of culture medium:

| glucose | 2.5-3.5 |
|---|---|
| maltose | 0.5-2 |
| corn flour | 0.3 |
| peptone | 0.5 |
| yeast powder | 0.5-1 |
| $KH_2PO_4$ | 0.2-0.4 |
| $MgSO_4 \cdot 7H_2O$ | 0.2-0.5 |
| vitamin $B_1$ | 0.05-0.1 |
| pH | 4.5—5.0 |

The rest is water.

(4) Filter the fermentation product of Cryptoporus volvatus (Peck) Schear in above-mentioned fermenter by normal filtration method to get mycelium and culture supernatant fluid. The filtration cake of Cryptoporus volvatus (Peck) Schear mycelium is dried at 80-90°C, crushed and sieved to give Cryptoporus volvatus (Peck) Schear powder of less than 60 mesh granules. Culture supernatant fluid is concentrated by rotate-vaporizing at 70-80°C until its density becomes 1.2-1.5 g/mL.

3. Clinic uses and dosage forms

In the present invention, Cryptoporus volvatus (Peck) Schear powder and culture supernatant concentrated fluid can be made respectively or combinedly into various medicines and health products.

For example, culture supernatant concentrated fluid can be heated to concentrate until it becomes creamy, and then add in Cryptoporus volvatus (Peck) Schear powder, which is blended with 5%-50% amylum and microcrystalline cellulose. Stir the mixture equably, dry, crush and sieve by 60 mesh sieves to give blended fermentation product.

Fermentation product of the present invention can be made into medicine to prevent and treat allergy, including I, II, III and IV type allergy, especially I type allergy of respiratory tract, derma, gastrointestinal tract and ophthalmopathy, such as bronchial asthma, allergic rhinitis, allergic dermatitis, drug allergy and food allergy, etc. This product can also be used as anti-irritability medicine, such as irritable diseases caused by pollen, acarid and mildew.

For convenience of using, the above-mentioned fermentation product can be made into various dosage forms by general methods, such as troche, capsule, pill, powder, granual, pastille, electuary, patch, syrup, mixture or aerosol.

Brief Description of the Drawings

Figure 1: effects of samples on pulmonary mechanical function of sensitized cavies after being attacked by antigen

Effects of fermentation product of cryptoporus sinensis Sheng H. Wu & M. Zang on airway resistance (RL) after being attacked by anaphylactic cavy antigen. Suppose airway resistance (RL) is 1005 before being attacked by antigen. Compared with the model group: *P<0.05, **P<0.001, ***P<0.001.

Figure 2: effects of samples on total leukocyte number in bronchial lung lavage solution of sensitized cavies after being attacked by antigen

Compared with the model group: *P<0.05, **P<0.001

T:      Extract of fermentation products of cryptoporus sinensis Sheng H. Wu & M. Zang

D:      dexamethasone sodium phosphate

K:      ketotifen

Figure 3: effects of samples on eosinophils in bronchial lung lavage solution of sensitized cavies after being attacked by antigen

Compared with the model group: *P<0.05, **P<0.001.

T:      extract of fermentation products of cryptoporus sinensis Sheng H. Wu & M. Zang
D:      dexamethasone sodium phosphate
K:      ketotifen

Description of the Invention

The following data of pharmacology, toxicology and clinic tests are given as specific illustrations of embodyments and uses of the invention.

(1) Effects on pulmonary mechanical function of sensitized cavies after being attacked by antigen.

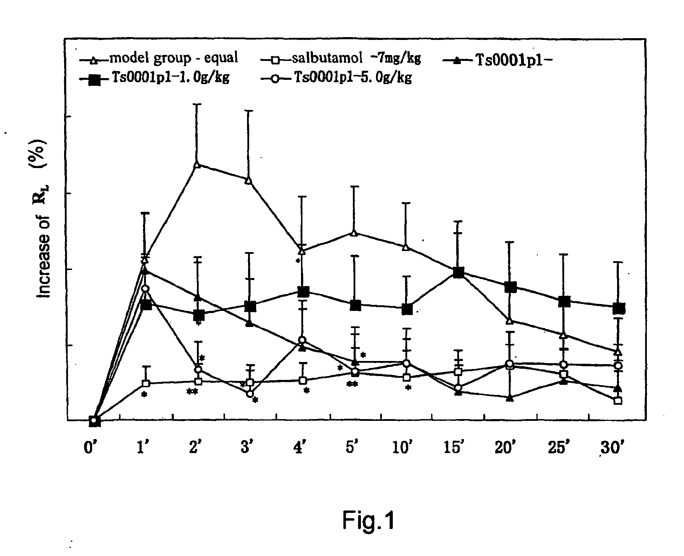

Each cavy is sensitized by being injected 10mg egg albumin into the muscles of its crus. 3-4 weeks later, antigen attack (1% egg albumin) is given and the caused changes of lung resistance ($R_L$) are observed to evaluate the activity of the samples. The results indicate that the fermentation products of cryptoporus sinensis Sheng H. Wu & M. Zang (sample Ts0001p1, 5.0g/kg×10d) have inhibition effect on increase of $R_L$ caused by antigen attack. There is significant statistical difference (P<0.05) at the time of 2min, 3min and 5min after antigen attack. $\beta_2$ receptor agonist salbutamol (7mg/kg×1d) as standard positive control, shows evident inhibition (P<0.05∼0.01) in all time phases during the 10min with the most serious airway resistant reaction after antigen attack. The results are illustrated in Figure 1.

(2) Changes of inflammatory cells in bronchial lung lavage solution of sensitized cavies after being attacked by antigen.

2% egg albumin gel solution is used to cause allergy and is subcutaneously injected (0.05mL each part) in ten parts of two retral soles, groin, waist, back, neck and armpits of each cavy. 0.5 mL 2% egg albumin gel solution is intraperitoneally injected at the same time. The medicine samples are injected since the tenth day after allergy has been caused. The testing samples of A2, B2, C2 or physiological saline solution (NS, 0.5mL/kg) or dexamethasone (DXM, 0.5mL/kg) are intraperitoneal injected respectively; A1, B1 (5g/kg), C1 (2.5g/kg) are administered by intragaster. The above testing samples are administered 10 days. A1: fermentation mycelium powder; B2: culture supernatant concentrated fluid; C1: sporophore powder; A2: water extract of fermentation mycelium; B2: fermentation concentrated fluid; C2: water extract of sporophore powder; dexamethasone sodium phosphate. Asthma as a chronic airway inflammatory disease is characterized by significant increase of eosinophils of bronchus and lung. Eosinophils can release tens of inflammatory substances, such as cytokines, leukotriene and prostapar etc., which may cause inflammation, edema, injury and thickening of airway, make narrow airway and therefore lead to dyspnea. Thus, eosinophil is considered as a target of screening drugs for relieving cough at present. The medicine, which can inhibit inflammation of airway eosinophils, would probably be used for preventing and treating asthma. In this test, substance B (fermentation concentrated fluid) shows good inhibition on eosinophils of bronchus and lung of sensitized cavies after being attacked by antigen (as described in Table 3)

Table 3:

| change of inflammatory cells in bronchial lung lavage solution of sensitized cavies after being attacked by antigen | | | | | | |
|---|---|---|---|---|---|---|
| Group | Dose | Administration | Leukocyte total number (Number/ mm$^3$) | Eosinophils (%) | Neutrophil (%) | Lymphocyte (%) |
| Normal | | | 87.6±61.32 | 0.20±0.4 | 4.3±3.5 | 97.5±3.5 |
| Model group | Equal NS | Ig | 360.7±219.1 | 34.30±18.8 | 23.4±11.9 | 43.1±20.8 |
| DXM | 0.5 mg/kg | Ip | 241.6±179.2 | 2.60±3.9*** | 28.9±16.6 | 68.4±18.4** |
| A2 | 0.5 ml/kg/d | Ip | 324.3±191.6 | 18.0±13.9* | 21.5±14.0 | 59.8±18.2 |
| B2 | 0.5 ml/kg/d | Ip | 299.1±151.0 | 6.18±5.40*** | 27.4±11.1 | 66.1±11.4** |
| C2 | 0.5 ml/kg/d | Ip | 263.4±138.0 | 12.57±11.3* | 36.6±6.19 | 50.9±10.0 |
| A1 | 5.0 ml/kg/d | Ig | 359.7±183.2 | 24.22±18.19 | 26.4±17.8 | 50.1±13.2 |
| B1 | 5.0 ml/kg/d | Ig | 224.0±107.4 | 8.40±5.79*** | 24.2±11.2 | 66.4±9.3** |
| C1 | 2.5 ml/kg/d | Ig | 309.4±240.3 | 9.00±7.37** | 23.6±14.0 | 67.3±11.1** |

(Compared with group of physiological saline solution *$P<0.05$,

**$P<0.01$,

***$P<0.001$)

(3) Effect of fermentation product of Cryptoporus volvatus (Peck) Schear with different dose on leukocyte total number and eosinophils in bronchial lung lavage solution of sensitized cavies after being attacked by antigen

75 SD cavies are divided into 8 groups and treated with 2% egg albumin gel solution to cause allergy. Subcutaneous injection is given into four soles of each cavy (0.1mL each part) and the operation is repeated for one time 10 days later. The cavies are administered with the medicine samples since the fourteenth day after allergy has been caused. The cavies are administered by intragaster method with extract of Cryptoporus volvatus (Peck) Schear fermentation product or with physiological saline solution (1mL/100g); or administered by intraperitoneal method with dexamethasone (0.5mg/kg); or administered by intragaster method with ketotifen (5mg/kg). All of the testing medicine samples are administered for 7 days. Since the fourteenth day after allergy has been caused, antigen attack is given 1 hour later after administration of medicine, for one time each day, altogether seven times. Among the groups, one control group is designed without antigen attack, but is injected with physiological saline solution instead. After attack, collect bronchial lung lavage solution and calculate leukocyte total number and eosinophils number. In model group which is attacked by antigen, compared to control group without antigen attack, the leukocyte total number and eosinophils number increases significantly which indicates that anaphylactic cavy antigen attack has obvious effect on allergic inflammation.

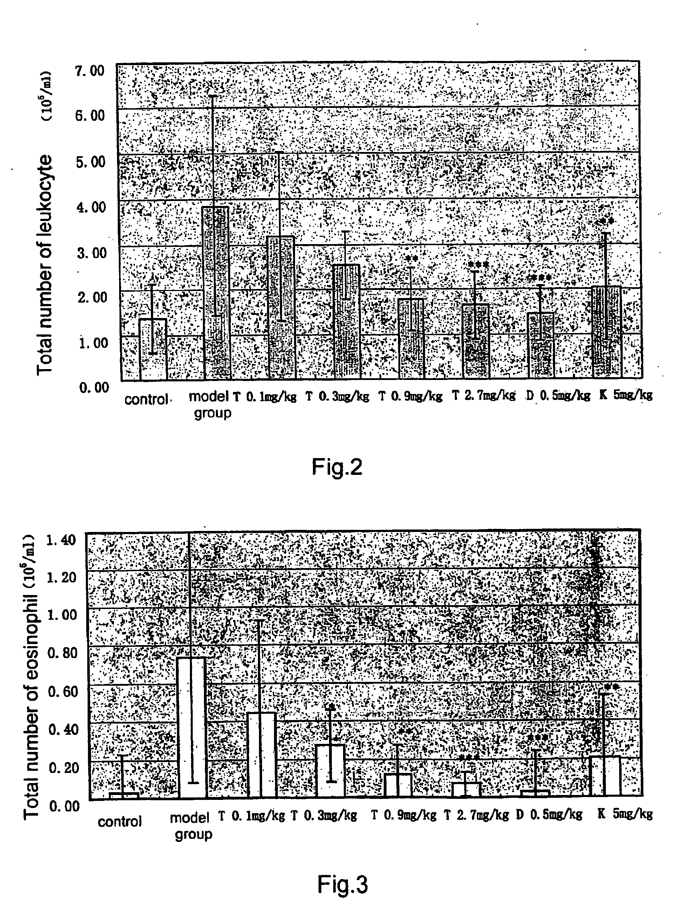

With dose dependence, cryptoporus sinensis Sheng H. Wu & M. Zang fermentation product shows inhibition on increase of eosinophils in bronchial lung lavage solution of sensitized cavies after being attacked by antigen. Inhibition on eosinophils $ID_{50}$ (95% confidence limits)=0.15 (0.11~0.21) g/kg. Inhibition on leukocyte total number $ID_{50}$ (95% confidence limits)=1.11 (0.73-1.70) g/kg. The results are shown in Figure 2 & 3.

(4) Effect on releasing leukotriene by polymorphonuclear granulocytes of cavies

The medicine samples are injected via muscle for 5 days continuously (2 times/day, 0.5mL/kg/time). Collect polymorphonuclear granulocytes in abdominal cavity of cavies and induce it by calcium ionopHore A23187 to release leukotriene. Test content of leukotriene $LTC_4$ by Leukotriene C4 EIA Kit. A: culture supernatant concentrated fluid deposited with 90% ethanol; B: deposit in 90% ethanol solution of culture supernatant concentrated fluid; C: culture supernatant concentrated fluid. These three medicine samples can evidently inhibit releasing of $LTC_4$ and have significant deference ($P<0.01$) compared with group of physiological saline solution. The result indicates these samples can effectively inhibit releasing of airway inflammatory medium (as described in Table 4).

Table 4:

| effects on releasing leukotriene of Polymorphonuclear granulocytes of cavies | | | |
|---|---|---|---|
| Group | Dose (ml/kg/d) | LTC4 (pg/ml) | Inhibiting rate (%) |
| Physiological saline solution | 1.0 | 90.63±16.36 | |
| A | 1.0 | 43.82±10.83** | 51.65 |
| B | 1.0 | 46.95±7.53** | 48.20 |
| C | 1.0 | 40.69±6.62** | 55.10 |

(Compared with group of physiological saline solution **$P<0.01$)

(5) Effects of elementarily separated substances on inflammatory cells in bronchial lung lavage solution of sensitized cavies after being attacked by antigen

2% egg albumin gel solution is used to cause allergy. The medicine samples are administered since the tenth day after allergy has been caused. The cavies are administered by introgaster method with this tested medicine sample or with physiological saline solution (10mL/kg); or administered by intraperitoneal method with dexamethasone (0.5mg/kg). All of the testing medicine samples are administered 10 days. A: total polysaccharide extracted from fermentation product; B: total alkaloids extracted from fermentation product; C: volatile oil extracted from fermentation product; D: organic acid extracted from fermentation product; E: water extract of fermentation product; positive control: dexamethasone. Sample A, namely, total polysaccharide extracted from fermentation product shows good inhibition on eosinophils inflammation of bronchus and lung of sensitized cavies after being attacked by antigen; and C, namely, volatile oil extracted from fermentation product also has strong effect (as described in Table 5).

Table 5:

| effects on inflammatory cells in bronchial lung lavage solution of sensitized cavies after being attacked by antigen | | | | | | |
|---|---|---|---|---|---|---|
| Group | Dose (ml/kg) | N | Leukocyte Total number (Number/mm3) | Eosinophils (%) | Neutrophil (%) | Lymphocyte (%) |
| Model group | Equal NS | 10 | 414.0±212.1 | 11.10±7.26 | 17.20±10.61 | 71.70±13.15 |
| DXM | 0.5 mg/kg | 10 | 330.5±254.3 | 0.00±0.00*** | 12.30±18.74 | 87.70±18.73** |
| A | 10 | 10 | 380.0±413.3 | 3.00±3.01** | 12.20±9.83 | 84.80±9.89** |
| B | 10 | 10 | 547.0±308.9 | 4.40±3.20* | 18.30±17.95 | 77.30±19.93 |
| C | 20 | 5 | 217.0±22.5 | 1.40±2.07** | 18.00±21.19 | 80.60±20.62 |
| D | 10 | 10 | 365.0±131.6 | 6.30±5.48 | 22.40±9.47 | 71.30±6.98 |
| E | 10 | 8 | 422.8±339.9 | 3.75±5.62* | 18.00±14.49 | 78.30±12.09 |

(Compared with group of physiological saline solution *$P<0.05$,

**$P<0.01$,

***$P<0.001$)

(6) Effects on cough reverberation of cavy caused by citric acid

Put cavies into a volume marked box to stabilize for 1 min, and then atomize 15% citric acid for 2 min by ultrasonic nebulizer. From then on, select the cavies that cough more than 10 times in 10 minutes. Divide the selected cavies into 5 groups in terms of their cough times, and then treat them respectively with testing medicine sample CVFS 0.3 g/kg, 0.9g/kg, 2.7g/kg, positive control codeine phosphate (10mg/kg), and the negative control group is administered by intragater method with 10ml·kg$^{-1}$ physiological saline solution (i.g), one time every day, altogether 3 days. 1 hour after the last administration of medicine, induce cough by above method and count the cough times in 10min from the beginning of atomization. The results indicate CVFS can evidently inhibit cough reverberation of cavy caused by citric acid. $ID_{50}$ (95% confidence limits) is 0.82 (0.64-1.04) g/kg. Inhibiting rate of codeine phosphate (10mg/kg) on cough reverberation of cavy caused by citric acid is 67.4% ($p<0.01$) as described in Table 6.

Table 6:

| inhibition of fermentation product on cough reverberation of cavy caused by citric acid ($\bar{x}\pm S$) | | | | |
|---|---|---|---|---|
| Group | N | Dose | Cough times ( in 10min) | Inhibition of cough times % |
| Physiological saline solution | 9 | 10ml·kg$^{-1}$ | 28.8±11.0 | |
| Codeine phosphate | 8 | 10mg·kg$^{-1}$ | 9.4 ±8.0** | 67.4 |
| Fermentation product | 8 | 0.3g·kg$^{-1}$ | 22.3±8.3 | 22.6 |
| Fermentation product | 8 | 0.9 g·kg$^{-1}$ | 11.1±8.4** | 61.5 |
| Fermentation product | 9 | 2.7 g·kg$^{-1}$ | 7.9 ±6.5** | 72.6 |

(Notes: Statistics: Student-Newman-Keuls, compared with group of physiological saline solution **P<0.01)

(7) Acute toxicity tests of fermentation product on mice

115 mice of Kunming stirp are administered by intragater method with extract of cryptoporus sinensis Sheng H. Wu & M. Zang fermentation product at dose of 45g/kg. The mice are fasted 12 hours before administration of medicine. After continuous administration for 8 days, no mice die and no any other toxicity are found. The maximum tolerance dose (MTD) of the mice is 45g/kg.

According to above data, it can be safely concluded that fermentation product of Cryptoporus volvatus (Peck) Schear in this invention has significant inhibition and effect on inflammatory cells and asthma, without toxic and side effect. Its preparation method is simple and fits for large-scale industrial production, thus the problem that pharmaceutical resources of Cryptoporus volvatus (Peck) Schear must be obtained from nature has been resolved.

The following examples are given as embodiments of the present invention.

Example 1

Slant culture method of Cryptoporus volvatus (Peck) Schear strain: transplant the Cryptoporus volvatus (Peck) Schear strain, which is kept at 4°C, to culture medium described as below via asepsis operation, and culture for 2-10 days at normal culture temperature of fungi.

Ingredients and weight contents (%) of culture medium:

| | |
|---|---|
| glucose | 1.5 |
| maltose | 0.6 |
| peptone | 0.2 |
| yeast powder | 1.4 |
| $KH_2PO_4$ | 0.3 |
| $MgSO_4 \cdot 7H_2O$ | 0.1 |
| vitamin B$_1$ | 0.03 |
| agar | 2.2 |
| The rest is water. | |
| pH5. | 0 |

Growth situation of thalli: Mycelial is dense, white, with radioactive growth at a fast speed.

Example 2

Slant culture method of Cryptoporus volvatus (Peck) Schear strain: transplant the Cryptoporus volvatus (peck) Schear strain, which is kept at 4°C, to culture medium described as below via asepsis operation, and culture for 8-10days at normal culture temperature of fungi.

Ingredients and weight contents (%) of culture medium:

| | |
|---|---|
| glucose | 1.5 |
| maltose | 0.6 |
| peptone | 0.04 |
| yeast powder | 0.3 |

(continued)

| KH$_2$PO$_4$ | 0.3 |
|---|---|
| MgSO$_4$ · 7H$_2$O | 0.1 |
| vitamin B$_1$ | 0.03 |
| agar | 2.2 |
| The rest is water. | |
| pH | 5.0 |

Growth situation of thalli: Mycelial is thin, slim, white, with radioactive growth at a relatively fast speed.

Example 3

Move the mycelial of activated Cryptoporus volvatus (Peck) Schear strain to 500mL triangle bottle containing 150mL culture medium as described below. Shaking-culture the mixture for 6 days on rotational bed at 28°C with a rotational velocity of 100rpm.
Ingredients of the basic liquid culture medium:

| glucose | 20 g |
|---|---|
| peptone | 10g |
| yeast powder | 3g |
| KH$_2$PO$_4$ | 1g |
| MgSO$_4$ | 0.5g |
| Water | 1000 ml |

pH of above culture medium is 5.5.
Growth situation of thalli: Culture fluid is clear; mycelial grows fastly; mycelial spheres are full of the whole culture medium of equal size.

Example 4

Take 20g fermentation mycelial powder, add in 400mL distilled water, boil and extract for 30min. After filtration, add another 200mL distilled water to the residue, then repeat the same steps. Mix the filtrate of the two times and add in 100mL culture supernatant fluid. Then put the mixture into dialysis bag of retention molecular weight 10,000 Dalton and dialyze 10mM pH6.0 buffer solution of HAc-NaAc 2000mL for three days to remove small molecular substances in the solution. Change the penetrated liquid 1 time each day.
Then concentrate the residue to 100mL. On animal test, the polysaccharide affects inflammatory cells in bronchial lung lavage solution of sensitized cavies after being attacked by antigen. (The results are described in Table 7.) 2% egg albumin gel solution is used to cause allergy and is subcutaneous injected (0.05mL each part) in ten parts of two retral soles, groin, waist, back, neck and armpits of each cavy. 0.5 mL 2% egg albumin gel solution is intraperitoneal injected at the same time. The samples are injected since the tenth day after allergy has been caused. The results show good inhibition on bronchial lung eosinophils of sensitized cavies after being attacked by antigen.

Table 7.

| Effects on inflammatory cells in bronchial lung lavage solution of sensitized cavies after being attacked by antigen | | | | | | |
|---|---|---|---|---|---|---|
| Group | Dose (ml/kg) | N | Leukocyte counts (Number/mm3) | Eosinophil (%) | Neutrophil (%) | Lymphocyte (%) |
| Model group | Equal NS | 10 | 414.0±212.1 | 11.10±7.26 | 17.20±10.61 | 71.70±13.15 |
| DXM | 0.5 mg/kg | 10 | 330.5±254.3 | 0.00±0.00*** | 12.30±18.74 | 87.70±18.73** |
| polysacchride | 10 | 10 | 380.0±413.3 | 3.00±3.01** | 12.20±9.83 | 84.80±9.89** |

(DXM is dexamethasone, compared with group of physiological saline solution **P<0.01,
***P<0.001)

Example 5

Preparation of capsules: boil the culture supernatant fluid to concentrate to semisolid, then add mycelium and 20% microcrystalline cellulose and mix in fast mixing granulator and granulate at 40 mesh pellet fabrication. Big granules can be granulated again after being dried. Put the granules into oven to dry below 80°C and ted at regular intervals until the moisture of granules is below 4%. To the 30 mesh whole granules, add 3% talcum powder. After mixing, pore it into capsules with 300mg/capsule. Pack after polishing, then sterilize under irradiation of Cobalt 60.

Example 6

Preparation of troche: boil culture supernatant fluid to concentrate to semisolid, then add mycelium, 34% microcrystalline cellulose and 17% foreclosed gel amylum and mix in fast mixing granulator and granulated at 10 mesh. Big granules can be granulated for the second time after being dried. Put the granules into oven at 80°C to dry and ted at regular intervals until the moisture of granules is 1-3%. To the 12 mesh whole granules, add 3% talcum powder. After mixing, press it into tablets with 500mg/tablet. Pack after coating color membrane, then sterilize under irradiation of Cobalt 60.

**Claims**

1.   A kind of fermentation product of Cryptoporus volvatus (Peck) Schear, it can be obtained by fermentation after culture of Cryptoporus volvatus (Peck) Schear; this product is a mixture of mycelium and culture supernatant fluid; its dry weight is 5-50g/1000mL; content of polysaccharide is 3~10%; content of volatile ether extract is 0.05~0.15%; content of total alkaloids is 0.03~0.25%.

2.   The fermentation product of claim 1, wherein Cryptoporus volvatus (Peck) Schear includes Cryptoporus sinensis Sheng H. Wu & M. Zang, Cryptoporus volvatus (Peck) Schear and other types of Cryptoporus volvatus (Peck) Schear.

3.   The fermentation product of claim 1, wherein said fermentation includes liquid fermentation and solid fermentation; fermentation product includes Cryptoporus volvatus (Peck) Schear liquid, mycelium of solid fermentation culture, culture supernatant fluid and various components which are extracted from above products.

4.   The fermentation product of claim 3, wherein said components include polysaccharide, volatile ether extract and alkaloids.

5.   A preparation method of the fermentation product of claim 1 or 3, Cryptoporus volvatus (Peck) Schear is cultured on slant culture medium by asepsis, then liquid or solid fermentation is done to get fermentation product.

6.   A preparation method of the fermentation product of claim 1 or 3, wherein this method includes activation of strain, shaking-culture, and liquid or solid fermentation culture.

7.   The method of claim 6, wherein the activation of strain is described as follows: transplant the strain of cryptoporus volvatus (Peck) Schear to the culture medium which contents more than 0.05% glucose, maltose, peptone, yeast powder, $KH_2PO_4$, $MgSO_4 \cdot 7H_2O$, vitamin $B_1$ and agar with pH3-7,and culture for 3-10 days at normal culture temperature of fungi.

8.   The method of claim 6, wherein the shaking-culture includes following steps: move the above-mentioned activated mycelial of Cryptoporus volvatus (Peck) Schear to the culture medium which contents more than 0.05% glucose, maltose, peptone, yeast powder, $KH_2PO_4$, $MgSO_4 \cdot 7H_2O$, vitamin $B_1$ and agar with pH3-7, and shaking-culture for 2-10 days on shaking bed at 20°C-30°C and shaking frequency of 50-200rpm.

9.   The method of claim 6, wherein the liquid fermentation culture includes the following steps: put the culture medium which contents more than 0.05% glucose, maltose, peptone, yeast powder, $KH_2PO_4$, $MgSO_4 \cdot 7H_2O$, vitamin $B_1$ and agar with pH3-7 into fermentation tank, add shaking-cultured Cryptoporus volvatus (Peck) Schear mycelial liquid after asepsis, and ferment for 1-10 days at ventilation quantity of 1: 0.1-1.0 (v/v/min), impeller stirring speed of less than 300rpm, tank pressure of more than $0.05kg/cm^2$ and temperature of 20-30 °C. Above culture medium can be compensated during fermentation.

10. Use of the fermentation product of claim 1 or 3, the fermentation product can be made into medicine to prevent and treat allergic diseases and cough.

11. The use of claim 10, wherein said allergic diseases include I, II, III and IV type allergic diseases.

12. The use of claim 11, wherein said allergic diseases include I type allergic diseases of respiratory tract, derma, gastrointestinal tract and ophthalmopathy.

13. The use of claim 12, wherein said allergic diseases include bronchial asthma, allergic rhinitis, allergic dermatitis, drug allergy, food allergy, gastroenteritis, conjunctivitis and keratitis.

14. Use of the fermentation product of claim 1 or 3, is to be made into medicine to prevent and treat irritability diseases and cough.

15. The use of claim 14, wherein said irritability diseases include irritabilities which is caused by pollen, acarid, mildew or dust.

16. Use of the fermentation product of claim 1 or 3, it can be made into troche, capsule, pill, powder, granual, pastille, electuary, patch, syrup, mixture or aerosol.

Fig.1

EP 1 422 292 A1

Fig.2

Fig.3

15

**EP 1 422 292 A1**

<table>
<tr><td colspan="2"><strong>INTERNATIONAL SEARCH REPORT</strong></td><td>International application No.<br><br>PCT/CN02/00508</td></tr>
</table>

| A. | CLASSIFICATION OF SUBJECT MATTER |
|---|---|

IPC⁷ C12N1/14 A61K35/84// C12N1/14,C12R 1:645

According to International Patent Classification (IPC) or to both national classification and IPC

| B. | FIELDS SEARCHED |
|---|---|

Minimum documentation searched (classification system followed by classification symbols)

IPC7 C07K, C12N, A61K

Documentation searched other than minimum documentation to the extent that such documents are included in the fields searched

Electronic data base consulted during the international search (name of data base and, where practicable, search terms used)

GenBank, EMBL,DDBJ,PDB, PIR,WPI

| C. | DOCUMENTS CONSIDERED TO BE RELEVANT |
|---|---|

| Category* | Citation of document, with indication, where appropriate, of the relevant passages | Relevant to claim No. |
|---|---|---|
| X | Wu Jinzhong etal,Fermentation Culture & Analysis of Compositions of Cryptoporus Volvatus(Peck) Schear,Journal of Fujian College of TCM,Feb 1999,9(1):33-36 | 1-16 |
| X | Wu Jinzhong et al,Analysis & Comparison of Compsitions Between Fermenttation Cultured & Wild Cryptoprus Volvatus,Journal of Fujian College of TCM,1997,7(2):21-26 | 1-16 |
| X | CN1058288C,HUA Qihong et al, 2000/11/08,See full-text | 1-16 |
| | | |
| | | |
| | | |

☐ Further documents are listed in the continuation of Box C.   ☒ See patent family annex.

| \* Special categories of cited documents: | "T" later document published after the international filing date or priority date and not in conflict with the application but cited to understand the principle or theory underlying the invention |
|---|---|
| "A" document defining the general state of the art which is notconsidered to be of particular relevance | |
| "E" earlier application or patent but published on or after the international filing date | "X" document of particular relevance; the claimed invention cannot be considered novel or cannot be considered to involve an inventive step when the document is taken alone |
| "L" document which may throw doubts on priority claim (S) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" document referring to an oral disclosure, use, exhibition or other means | |
| "P" document published prior to the international filing date but later than the priority date claimed | "&" document member of the same patent family |

| Date of the actual completion of the international search<br><br>22.Oct.2002(22.10.02) | Date of mailing of the international search report<br><br>16 JAN 2003 (16.01.03) |
|---|---|
| Name and mailing address of the ISA/CN<br>6 Xitucheng Rd., Jimen Bridge, Haidian District,<br>100088 Beijing, China<br>Facsimile No. **86-10-62019451** | Authorized officer<br><br>WangPengFei<br><br>Telephone No. **86-10-62093145** |

Form PCT/ISA /210 (second sheet) (July 1998)